# EUROPEAN PATENT APPLICATION

(11) **EP 0 888 756 A2**
(43) Date of publication of application: **07.01.1999**
(21) Application number: 98111095.0
(22) Date of filing: 17.06.1998
(51) Int. Cl.: A61F 2/00

(54) **Plug for hernioplasty with monofilament polypropylene mesh**

(30) Priority: 18.06.1997 CH 1481/97
(71) Applicant: Herniamesh S.r.l., 27100 Pavia (IT)
(72) Inventor: Trabucco, Ermanno, Greatcrech Long Island NY 11020 (US)
(74) Representative: Moretti, Francesco, Dr.

(57) **Abstract**

The prosthetic plug comprises a base (1) of circular or rectangular shape made of monofilament macroporous polypropylene mesh of surgical quality of rigid type with controlled memory for the tension free suturless positioning of said plug, and a flattened blunted apex (2) with rounded shape (2a) made of a preformed mesh of surgical quality with surface memory for its introduction and obstruction inside the wall defect. Unlike the plugs of the prior art that cause tension or sting and tickle under the skin because of their point after the implant, or like the cylindrical plugs requiring an awkward preparation during the operation and are difficult to be accomplished without tension, the plug of the invention allows tension free implants with pre-peritoneal approach for relapsing cruroinguinal herniae, for front repair of crural herniae and with front approach repair of big relapsing inguinal herniae. The plug can be more particularly applied in hernioplasty surgery, more particularly in the inguinal surgery, with dramatic reduction of failures and relapses.

## Description

The present invention refers to a plug for surgical treatment and cure of hernioplasty, more particularly of relapsing inguinal herniae. The prosthesis in polypropilene mesh is the material preferred by sergeants for the modern hernia surgery.

Polypropylene mesh has high tensile resistance, is biologically stable, causes a quick fibroplastic proliferation leading to a more efficient deposition of collagen while up to now there is no evidence of inducing allergic responses or carcinogenic events. Polypropylene mesh is very resistant to infections just for its monofilament characteristic, but the disadvantages of this material are memory and thermolability at the temperature of autoclave sterilization, as well as the tendency to deformation some time after implant. After autoclave treatment at 126°C the mesh undergoes a deformation noting that the melting point of polypropylene is about 165°C.

A mesh with memory is difficult to implant, because it has a tendency to crumple and to lay in an incorrect way, and these incorrect positions may occur either during or after implant, causing dead spaces, serum formation, mesh migration and relapses.

A polypropylene mesh retaining a surface memory and resisting to this tendency to crumple is called controlled memory (CM) mesh. A polypropylene mesh resisting to deformation after sterilization in autoclave and keeping unaltered its characteristics after implant is called surgical quality (SQ) mesh.

These and other considerations are the basis of the operative choice of this invention. In order to obtain surgical quality and controlled memory polypropylene before weaving undergoes special processes in absence of chemical treatments. From the first clinical tests it seems that these meshes may actually be innovative.

According to the above mentioned principles different types of meshes were carried out; those called type 1 and type 2 are preformed rigid and semi-rigid meshes, mainly suitable for tension free suturless hernioplasty.

The indication to use a double mesh was dictated by the need to have at disposal an article that has no tendency to crumple during the implant maneuvres; this mesh was initially accomplished in a handicraft way by joining two polypropylene meshes, preforming them, welding the edges and sterilizing thereafter with gas. A double mesh so obtained has however the disadvantage of a dead space between the two meshes, that is a barrier slowing down the fibroplastic infiltration causing the prostheses to be less resistant to infections.

The preformed controlled surgical quality mesh of the present invention is of a single layer and has characteristics of elasticity and resistance higher than the double layer one, its surface memory allows to obtain an implant that does not crumple during the surgical maneuvres.

The type 3 mesh or macroporous mesh is used to make plugs of the type called T1. These medical plugs having the shape of a dart are particularly adapted for treatments at the external inguinal ring. The plug after application opens making a better protection in the space behind the fascia trasversalis, so as to prevent relapse of sack herniation. The mesh of this type is suitable also for treatment of laparocele of inguinal hernioplasty with Lichtenstein or Rives technique.

The type 4 mesh is bielastic preformed with large pores and can be shaped according to the need, being suitable for Stoppa or Wantz technique. In our opinion polypropylene is more suitable than mersilene disclosed in the original techniques of these authors as it is handier and more bio-compatible.

The type 5 mesh is very porous and suitable for laparoscopy. The plugs being the subject of the present invention are made using mesh type 3 for the base and mesh type 1 or 2 for apex, and are suitable for correction of inguinal and cruroinguinal relapses or for crural hernia.

The present invention refers to a plug with round or square base of variable width for treatments of the internal inguinal ring in surgery and treatment of hernioplasty.

The plugs of the prior art are generally shaped in the form of a dart or arrow point with consequent drawbacks, either because they cause tension or because even when they are implanted with the free tension suturless technique, once implanted, as they are pointed and placed under cutis, they sting or tickle causing trouble in the patient. Also the cylindrical plugs without point do not succeed to remove entirely tensions, although one takes measures of a full or empty core to create dead spaces where tensions are released, and moreover these plugs are awkward in the preparation during surgery.

In order to remove the drawbacks of prior art we carried out a plug comprising a flat rounded point which is introduced free inside the wall defect and a base consisting of a mesh of proper shape and size according to the type of operation, for its implant and positioning without tension.

More particularly the present invention refers to a plug for surgery and treatment of hernioplasty made of polypropylene mesh with controlled memory CM and surgical quality SQ, characterized by the fact of comprising a base made of macroporous mesh and a rounded point of preformed mesh with surface memory joined together by stitches.

In some cases an auxiliary mesh of round or rectangular shape may be coupled to the plug base so as to reach in this case the size of 10x14 cm. The flat rounded point may be accomplished according to the type of use with polypropylene mesh of type 1 or 2, that is with preformed rigid or semi-rigid mesh.

A detailed description of some non limiting embodiments of the present invention is given hereinafter with reference to the figures of the accompanying drawings however without departing from the general scope of the invention.
Figure 1 shows the prosthetic plug of the invention provided with a round base and a rounded flat blunted apex;
Figure 1a shows the plug of Figure 1 joined with an auxiliary rectangular mesh;
Figure 2 shows the prosthetic plug of the invention in a bottom view showing the stitches or sutures on the circular base; and
Figure 3 shows the prosthetic plug of the invention in a tension free repair implant of a cruro- inguinal hernia.

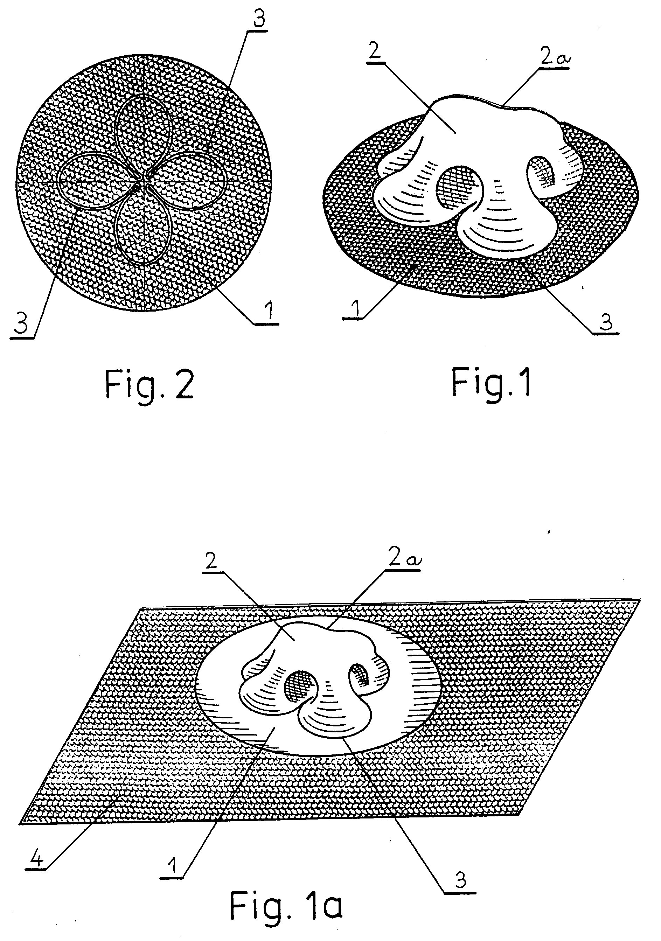

The prosthetic plug for surgery and treatment of hernioplasty made of polypropylene mesh with controlled memory CM and surgical quality SQ, is characterized by comprising a base 1 of macroporous mesh and a point 2 rounded as in 2a of preformed mesh with surface memory joined together by stitches 3 (Figs. 1, 1a to 3).

In a particular embodiment referred to as plug T2, the prosthetic plug uses for the base 1 a microporous mesh of type 3 and a type 1 mesh for the apex 2, 2a (Figs. 1, 1a). In this embodiment the round base has preferably a diameter of 5 cm and is used in repairing crural herniae and circumscribed cruroinguinal relapses.

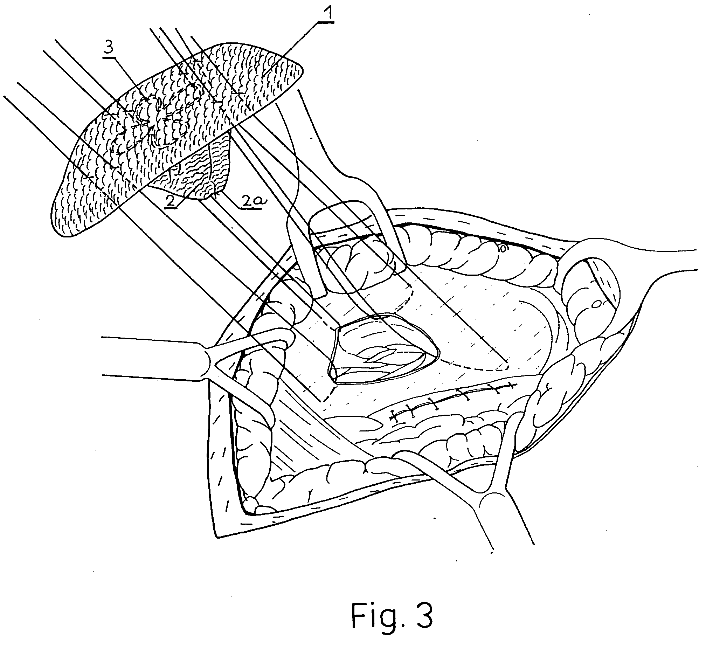

In this kind of operation four U shaped stitches are inserted in the four quadrants of the hernia orifice of the cruroinguinal back wall and then are inserted into the plug base; the four stitches are tied up and the plug point slips tension free inside the hernial defect (Fig. 3).

This plug replaces efficiently the cylindrical prosthetic plug in repairing relapsing inguinal herniae with front approach.

In case of repair through the front route of the crural hernia the apex may be of 3.5 cm of diameter.

Another embodiment consists of the prosthetic plug of type T3, which uses too for the base 1 a polypropylene macroporous mesh of type 3 and for the point 2 preformed and rounded at 2a a type 1 mesh, but unlike plug T2 this plug is arranged on an auxiliary mesh 4 much bigger and of square or rectangular shape (Fig. 1a) reaching the size of 10x14 cm. This combination is used when fascia trasversalis needs to be reinforced and is used through the preperitoneal route for big inguinal relapsing herniae.

The meshes making the plug are all made of monofilament polypropylene and are previously treated so as to increase heat resistance and decrease memory; in other words they are provided with controlled memory, namely they keep the flat configuration of the sterile article for a more efficient and safer repair of hernia; they are all meshes with surgical quality, namely treated after weaving to increase thermostability and with thin texture to increase the fibroplastic infiltration. The single layer mesh of the plug base has a stiffness suitable for tension free suturless hernioplasty, The plugs may be sterilized again with ethylene oxide.

In case of relapsing inguinal herniae Lichtenstein noted that in 95% of cases the relapsing herniae are due to a single wall defect, almost always at the inner corner or at the deep inguinal ring. For 75% of cases moreover the relapsing hernia does not exceed a diameter of 4 cm.

In case when the relapsing hernia is single and lower than 4 cm, Lichtenstein proposes to apply through the inguinal route a prosthetic plug. The operation may be carried out with local anaesthetic. It is very important before the operation to locate the exact point of hernia in both upright and laying position of the patient and mark it with a indelible marking pen. The cutaneous cut extended for 4-5 cm is centered on the top of the hernia tumefaction; having found the elements of the funiculus the hernial sack is isolated; the identification of hernia may be made easier by requesting the patient to do some cough.

Usually the sack is in the subcutaneous tissue, but sometimes it can be under the aponeurosis of the external oblique muscle. The sack is freed from the fascia that formes at this level a solid ring formed by cicatricial tissue and is then introflexed inside the abdomen. With the forefinger introduced through the hernial door, the condition of the remaining back wall of the inguinal channel is explored; if there are other herniae or if the wall should result weakened also in other points, it is advisable to choose a different type of repair.

Then the prosthetic plug of the invention, identical to the plug of the crural herniae, is introduced inside the wall defect that must result fully obstructed; the base is then sutured to the surrounding fascia with spaced stitches for example of prolene. With this tension free prosthetic hernioplasty technique it is dramatically reduced the number of failures and relapses.

The prosthetic plug of the present invention is particularly adapted in surgery and treatment of hernioplasty, more particularly for front repair of crural, inguinal and relapsing cruroinguinal herniae, as well as for treatment at the internal inguinal ring.

## Claims

1. Plug for surgery and treatment of hernioplasty made of polypropylene mesh with controlled memory (CM) and surgical quality (SQ), characterized by comprising a base (1) made of macroporous mesh and a point (2) rounded (2a) made of preformed mesh with surfaces memory joined together by stitches (3).

2. Hernioplasty plug according to claim 1, characterized in that a rectangular auxiliary mesh (4) is coupled to the base (1).

3. Hernioplasty plug according to claim 2, characterized in that the rectangular auxiliary mesh (4) has a size of 10 x 14 cm.

4. Hernioplasty plug according to claim 1 characterized in that the base (1) has a circular shape.

5. Hernioplasty plug according to any of claims 1 to 4 characterized in that the point (2) rounded (2a) is made of rigid mesh suitable for tension free suturless implant.

6. Hernioplasty plug according to any of claims 1 to 4 characterized in that the point (2) rounded (2a) is made of semi-rigid mesh.

7. Hernioplasty plug according to any of claims 1 to 6 characterized in that the point has a size between 3.5 and 4 cm diameter.

8. Use of the plug according to any of claims 1 to 7, in surgery and treatment of hernioplasty, more particularly for the front repair of crural, inguinal and relapsing cruroinguinal herniae and for treatment at the internal inguinal ring.
